# DEMANDE DE BREVET EUROPEEN

(11) **EP 1 611 921 A1**
(43) Date de publication de la demande: **04.01.2006**
(21) Numéro de dépôt: 05291345.6
(22) Date de dépôt: 23.06.2005
(51) Int. Cl.: A61M 39/26, A61M 16/08, F16L 37/60

(54) **Prise médicale destinée à la mise en oeuvre de fluides médicaux**

(30) Priorité: 29.06.2004 FR 0407322
(71) Demandeur: Yog Industries, 74460 Marnaz (FR)
(72) Inventeur: Goy, Lucien, 74300 THYEZ (FR)
(74) Mandataire: Gasquet, Denis

(57) **Abrégé**

Prise médicale (1) solidaire d'une poutre de distribution, destinée à la mise en oeuvre de fluides médicaux afin d'effectuer des branchements rapides d'une fiche d'un flexible d'un appareil, sans risque d'inversion des fluides, du type comprenant une embase de prise murale (2) constituant un point de sortie ou d'entrée dans le circuit de distribution et destinée à être raccordée sur une tuyauterie (3), notamment par brasure, laquelle embase (2) se prolonge par un corps d'embout (4), l'ensemble renfermant une partie fonctionnelle (5) formée par un clapet d'embase (6) et d'un filtre (7), spécifiques et adaptés aux fluides, caractérisée en ce que ladite prise (1) est réalisée en deux parties distinctes, dont une première est formée par l'embase de prise murale (2) fixée à demeure à la poutre de distribution et par rapport à la tuyauterie d'alimentation, et dont une seconde est formée par le corps d'embout (4) renfermant la partie fonctionnelle (5) spécifique aux fluides à distribuer, l'une et l'autre des parties comportant des moyens de raccordement complémentaires, de manière à permettre une interchangeabilité de la seconde partie sans intervention sur la première, pour remplacement ou en fonction du fluide à distribuer.

## Description

La présente invention concerne une prise destinée à la mise en oeuvre de fluides médicaux, à raccorder à un système de distribution, sur laquelle un opérateur peut brancher ou débrancher un appareil destiné à alimenter en fluide médical spécifique.

C'est ainsi qu'à partir d'une poutre de distribution plusieurs prises médicales pourront être fixées sur celle-ci et alimenter des appareils d'anesthésie, ventilateurs pulmonaires ou tout autre dispositif médical distribuant soit de l'oxygène ; du protoxyde d'azote ; de l'air respirable ; du dioxyde de carbone ; mélange oxygène - protoxyde d'azote ; de l'air pour les instruments chirurgicaux, vide (aspiration), sachant que tout raccordement erroné peut mettre la vie du patient en danger.

Il est donc vivement recommandé et d'ailleurs imposé par les normes de n'utiliser qu'un seul type de prise murale par gaz, et de normaliser la connectique.

Les prises médicales de ce type comprennent une embase de prise murale constituant un point de sortie ou d'entrée dans le circuit de distribution et destinée à être raccordée sur une tuyauterie, notamment par brasure, laquelle embase se prolonge par un corps d'embout, l'ensemble renfermant une partie fonctionnelle formée par un clapet d'embase et un filtre spécifique adapté à un fluide donné.

Ces prises sont conçues de manière monobloc en formant l'embase et le corps d'un seul tenant.

Un inconvénient majeur découle d'une telle conception, qui réside dans le fait que lorsqu'une prise devient défectueuse, il est nécessaire de changer l'ensemble de celle-ci.

Pour cela, il est nécessaire d'effectuer une coupure du réseau de fluide, puis de débraser et de rebraser une nouvelle prise.

Il est également nécessaire de protéger la partie fonctionnelle formée par le clapet et le filtre avant de rebraser la nouvelle prise.

Un autre inconvénient réside dans le fait que l'embase et le corps ainsi réalisés d'une seule pièce est d'une fabrication compliquée et donc onéreuse.

Enfin, sur le plan de la gestion du matériel, il est nécessaire d'avoir autant de prises qu'il y a de fluides à distribuer, la partie fonctionnelle étant différente selon le cas.

La présente invention a pour objet de remédier à ces différents inconvénients et concerne à cet effet une prise médicale solidaire d'une poutre de distribution, destinée à la mise en oeuvre de fluides médicaux afin d'effectuer des branchements - des branchements rapides d'une fiche d'un flexible d'un appareil, sans risque d'inversion des fluides, du type comprenant une embase de prise murale constituant un point de sortie ou d'entrée dans le circuit de distribution et destinée à être raccordée sur une tuyauterie, notamment par brasure, laquelle embase se prolonge par un corps d'embout, l'ensemble renfermant une partie fonctionnelle formée par un clapet d'embase et d'un filtre, spécifiques et adaptés aux fluides, caractérisée en ce que ladite prise est réalisée en deux parties distinctes, dont une première est formée par l'embase de prise murale fixée à demeure à la poutre de distribution et par rapport à la tuyauterie d'alimentation, et dont une seconde est formée par le corps d'embout renfermant la partie fonctionnelle spécifique aux fluides à distribuer, l'une et l'autre des parties comportant des moyens de raccordement complémentaires, de manière à permettre une interchangeabilité de la seconde partie sans intervention sur la première, pour remplacement ou en fonction du fluide à distribuer.

Il est ainsi permis de simplifier la fabrication de ce type de prise et de diminuer le nombre de références.

Ceci tient au fait qu'il est possible de changer le corps d'embout détérioré par les branchements et débranchements successifs sans avoir à dessouder puis ressouder une autre prise complète.

Ainsi, lors de la réalisation du réseau, on n'approvisionne que la partie embase à braser sur la tuyauterie sans risque de détériorer la partie fonctionnelle renfermée par le corps qui sera montée ultérieurement et en fonction du fluide à distribuer, les embases demeurant toujours identiques.

L'embase est ainsi considérée comme un simple élément de tuyauterie conditionné de manière standard, alors que les corps renfermant la partie fonctionnelle deviennent des éléments nobles conditionnés distinctement à l'unité et utilisés à la demande.

La présente invention concerne également les caractéristiques qui ressortiront au cours de la description qui va suivre, et qui devront être considérées isolément ou selon toutes leurs combinaisons techniques possibles.

Cette description donnée à titre d'exemple non limitatif, fera mieux comprendre comment l'invention peut être réalisée, en référence aux dessins annexés sur lesquels :

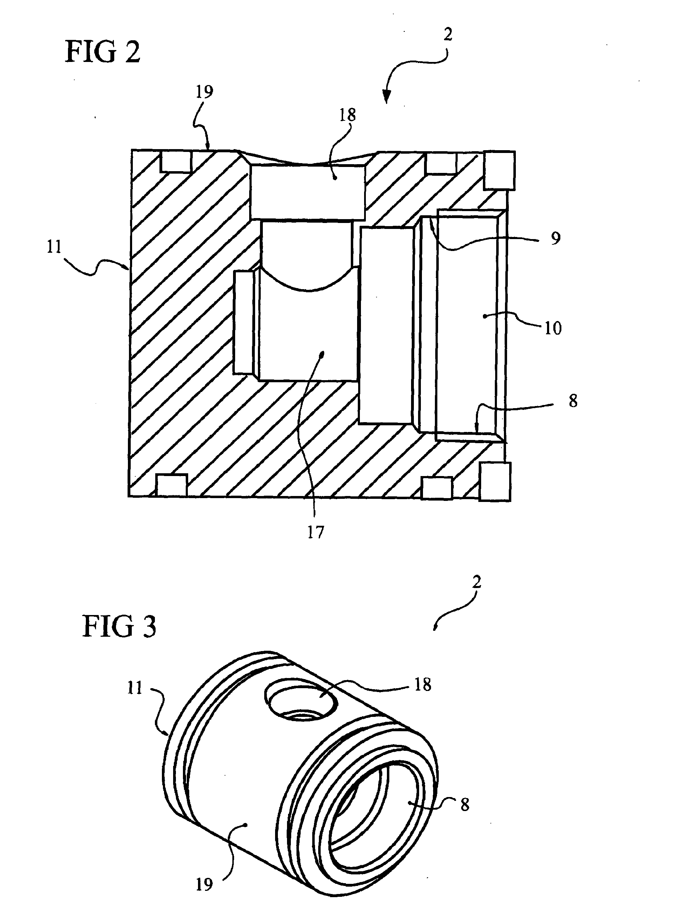

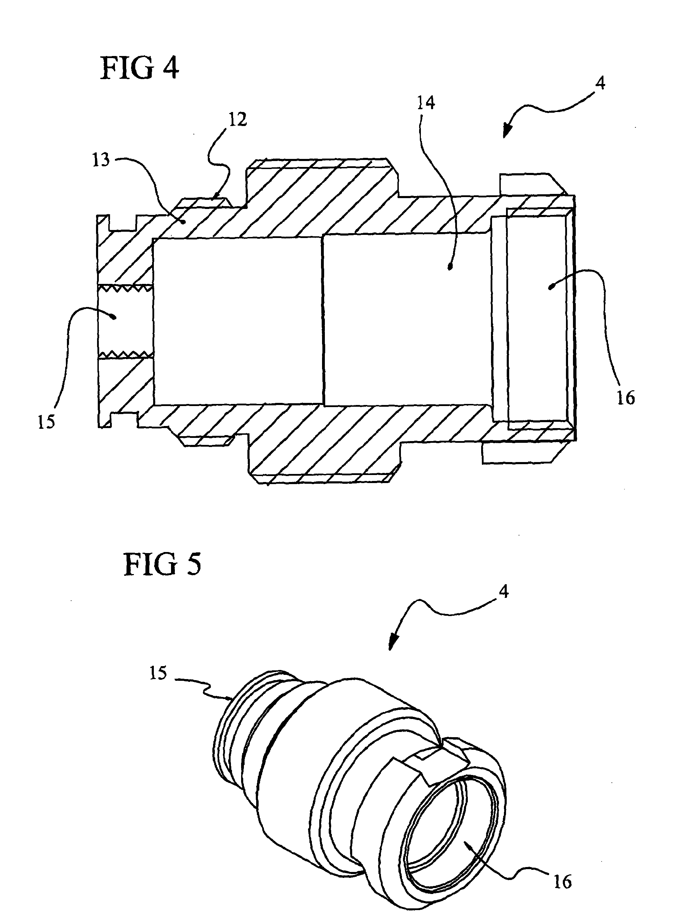

Les figures 1 à 5 illustrent un premier mode de réalisation.
La figure 1 est une vue schématique en coupe selon le plan de symétrie générale, d'une prise médicale selon l'invention comportant une embase, un corps d'embout rapporté à l'intérieur duquel est disposée une partie fonctionnelle spécifique.
La figure 2 est une vue en coupe d'une embase constitutive d'une prise selon la figure 1.
La figure 3 est une vue en perspective d'une embase selon la figure 2, à échelle réduite.
La figure 4 est une vue en coupe d'un corps d'embout constitutif d'une prise selon la figure 1.
La figure 5 est une vue en perspective d'une embase selon la figure 4.
Les figures 6 à 8 illustrent un deuxième mode de réalisation.

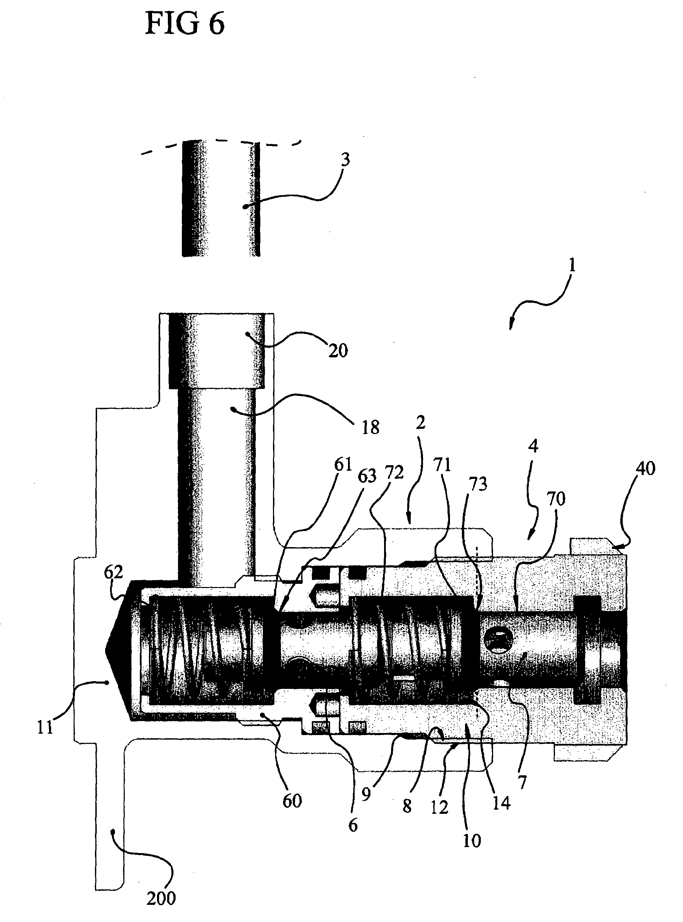

La figure 6 est une vue schématique en coupe selon le plan de symétrie générale.

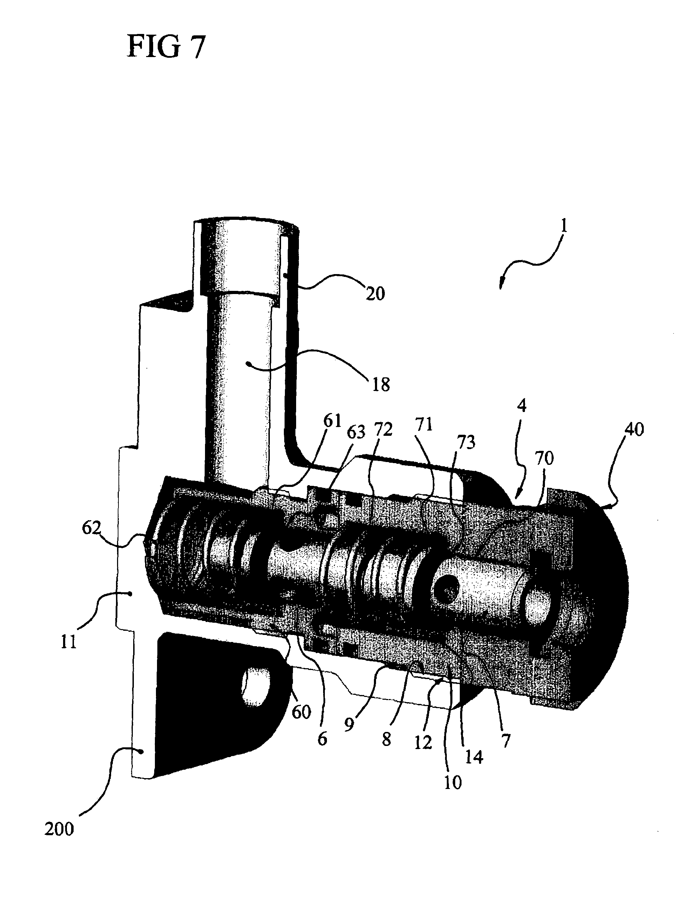

La figure 7 est une vue en perspective et en coupe longitudinale, de la prise dans sa position de fermeture, quand le raccordement correspondant de la canalisation d'alimentation au patient n'est pas raccordé sur ladite prise.

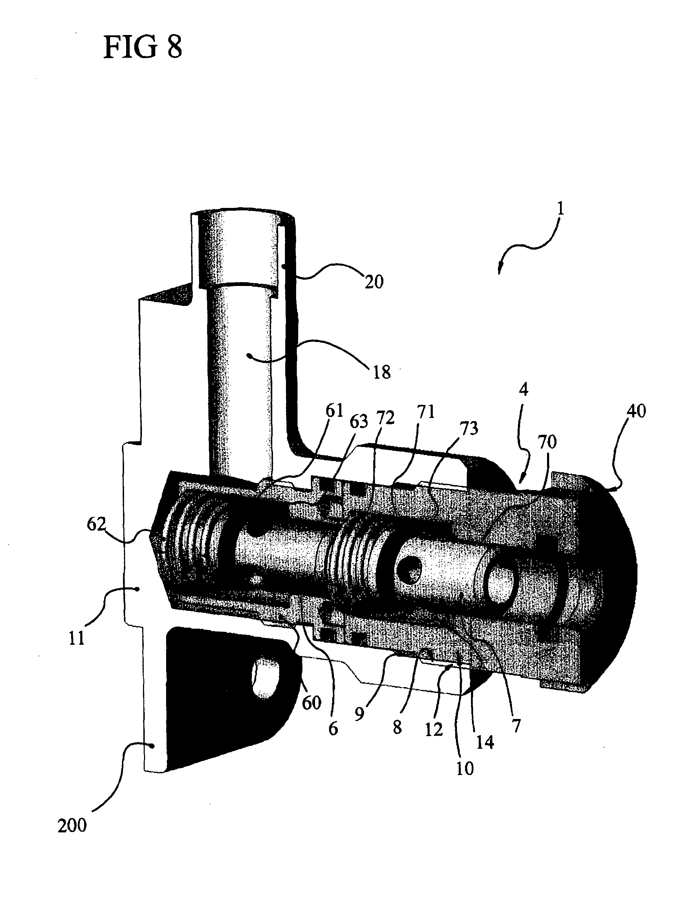

La figure 8 est une vue en perspective et en coupe longitudinale, de la prise dans sa position d'ouverture quand le raccordement correspondant de la canalisation d'alimentation au patient est raccordé sur ladite prise (ledit raccordement n'étant pas représenté).

On décrira tout d'abord le premier mode de réalisation.

La prise médicale désignée dans son ensemble par la référence (1), est solidaire du mur (100) et est avantageusement logé dans une poutre de distribution (non représentée) destinée à la mise en oeuvre de fluides médicaux afin d'effectuer des branchements - rapides d'un raccord d'un flexible ou canalisation de distribution au patient d'un appareil médical sans risque d'inversion des fluides.

La prise (1) est constituée par une embase de prise murale (2) constituant un point de sortie ou d'entrée dans le circuit de distribution et destinée à être raccordée sur une tuyauterie (3) notamment par brasure, laquelle embase (2) se prolonge vers l'avant par un corps d'embout (4), l'ensemble renfermant une partie fonctionnelle (5) formée par un clapet d'embase (6) et un filtre avec clapet (7), spécifiques adaptés aux fluides.

Selon l'invention, la prise (1) est réalisée en deux parties distinctes, dont une première partie est formée par l'embase de prise murale (2) fixée à demeure sur le mur (100) et par rapport à la tuyauterie d'alimentation, et dont une seconde partie est formée par le corps d'embout (4) comprenant la partie fonctionnelle (5) spécifique aux fluides à distribuer, l'une et l'autre des parties comportant des moyens de raccordement complémentaires, de manière à permettre une interchangeabilité de la seconde partie sans intervention sur la première, pour remplacement ou en fonction du fluide à distribuer.

Préférentiellement, ces moyens de raccordement complémentaires sont constitués d'une part par un filetage interne avant (8) réalisé sur l'alésage (9) d'une extrémité ouverte (10) de l'embase (2) opposée à une extrémité (11) formant un fond, et d'autre part par un filetage externe arrière (12) réalisée à une extrémité arrière (13) du corps d'embout (4), apte à coopérer en vissage avec le filetage (8) de l'embase (2).

Plus précisément, le corps d'embout (4) est traversé de part en part par un logement axial (14) présentant des diamètres étagés, dont une extrémité (15) dirigée vers l'embase (2) est d'un diamètre réduit par rapport au logement (14) et filetée intérieurement pour permettre le vissage d'un clapet choisi (6) et dont l'autre extrémité (16) opposée est de diamètre élargi par rapport audit logement (14) et filetée intérieurement pour permettre le vissage d'un filtre avec clapet choisi (7), pour constituer ainsi un sous-ensemble spécifique et indépendant de l'embase (2), ne constituant qu'un élément de tuyauterie standard et inamovible.

En ce qui concerne l'embase (2), celle-ci forme un logement axial borgne (17) dans lequel débouche un trou radial(18) traversant radialement la paroi périphérique (19) de l'embase (2) et sur lequel trou (18) est rapportée par brasage une portion de tuyauterie (20), destinée à être raccordée ultérieurement à un circuit de fluide (3).

Préférentiellement, l'embase (2) est en laiton, tandis que la portion de tuyauterie rapportée (1), est en cuivre, et liée à l'embase par brasage.

L'invention concerne également un procédé de fabrication d'une embase (2) et d'un corps d'embout (4) tels qu'ils viennent d'être décrits.

En ce qui concerne l'embase (2), les étapes d'usinage sont les suivantes :
- décolletage pour l'obtention d'une pièce de révolution
- perçage du trou radial (18)
- brasage de la portion de tuyau (20) sur l'embase (2)
- décapage de la pièce ainsi obtenue
- conditionnement

En ce qui concerne le corps d'embout (4), celui-ci est réalisé selon les étapes d'usinage suivantes :
- usinage pour l'obtention d'une pièce de révolution
- chromage
- montage d'un clapet (6) et d'un filtre spécifique (7)
- conditionnement

L'obtention de la pièce de révolution constituant l'embout (4) pourrait être obtenue par exemple par tournage et fraisage des deux côtés de la pièce.

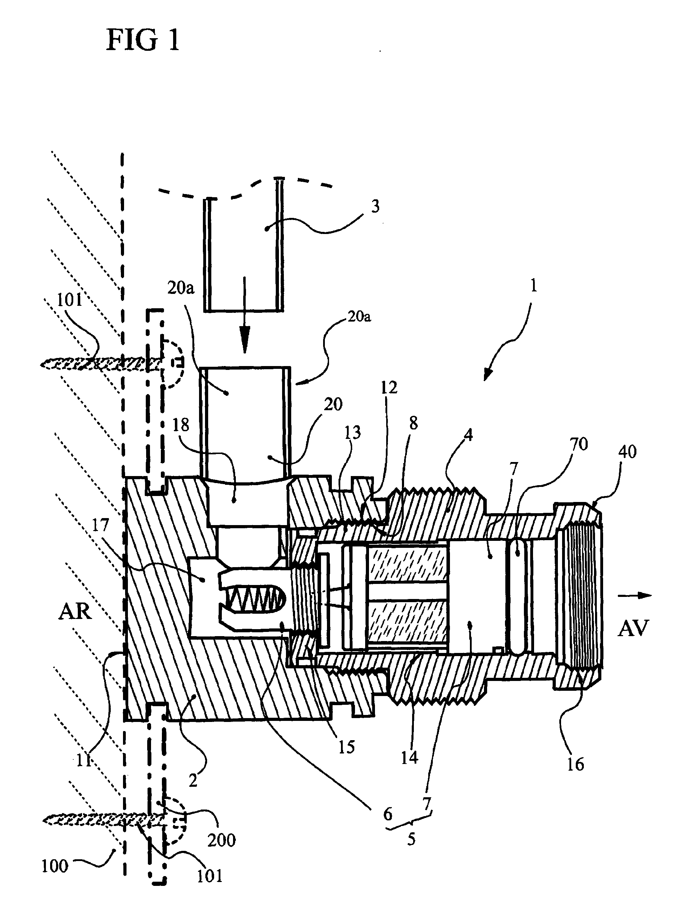

Selon le premier mode de réalisation la fixation de l'embase murale (2) sur le mur correspondant (100) est réalisée grâce à une plaque de base (200) amovible in dépendante de l'embase, qui est engagée dans la rainure périphérique (200) tandis qu'elle est fixée au mur grâce à un ensemble de vis de fixation (101), tel que cela est illustré entraits pointillés à la figure 1.

Selon le deuxième mode de réalisation, la plaque de base (200) est solidaire de l'embase murale (2), de même que la portion de tuyauterie (20), pour former une seule et même pièce.

Dans la description qui va suivre du deuxième mode de réalisation, les pièces et éléments similaires au premier mode de réalisation porteront les mêmes références.

Ainsi, comme précédemment, la prise médicale (1) est réalisée en deux parties distinctes, à savoir une première partie formée par l'embase de prise murale (2) fixée à demeure au mur correspondant et par rapport à la tuyauterie d'alimentation, et une seconde partie formée par le corps d'embout (4) l'une et l'autre des parties comportant des moyens de raccordement complémentaires, de manière à permettre une interchangeabilité de la seconde partie sans intervention sur la première, pour remplacement ou en fonction du fluide à distribuer.

Comme précédemment le corps d'embout (4) est fixé de façon amovible sur l'embase murale correspondante (2) par vissage.

Selon ce deuxième mode de réalisation, l'embase murale (2) constituant un point d'entrée dans le circuit de distribution et destinée à être raccordée sur une tuyauterie (3).

La prise médicale (1) avec son embase murale (2) prolongé par le corps d'embout (4) comprend selon son axe général un clapet d'embase (6) et un filtre-clapet (7), spécifiques adaptés aux fluides.

Les moyens de raccordement permettant de fixer le corps d'embout (4) sur l'embase murale (2) sont constitués comme selon le premier mode de réalisation d'une part par un filetage interne (8) réalisé sur l'alésage (9) d'une extrémité avant ouverte (10) de l'embase (2) opposée à une extrémité arrière (11) formant un fond, et d'autre part par un filetage externe (12) réalisée sur le corps d'embout (4), apte à coopérer en vissage avec le filetage (8) de l'embase murale (2).

On ajoutera que le corps d'embout (4) est traversé de part en part par un logement axial (14) destiné à recevoir le filtre-clapet (7). On notera que ce dernier est constitué par une cartouche (70) comprenant un joint torique d'étanchéité (71), tandis que la cartouche est sollicitée vers l'avant en position de fermeture grâce à un ressort de compression conique (72), ladite position de fermeture étant celle ou le joint torique d'étanchéité (71) est en appui sur son siège d'appui (73).

Selon le deuxième mode de réalisation, on notera que le clapet d'embase (6) est logé dans une cartouche d'embase (60) qui comprend un joint torique d'étanchéité (61), tandis qu'il est sollicité vers l'avant en position de fermeture grâce à un ressort de compression conique (62), ladite position de fermeture étant celle ou le joint torique d'étanchéité (61) est en appui sur son siège d'appui (63).

Bien entendu l'extrémité avant (40) du corps d'embase (4) est pourvu de moyens de connexion pour recevoir le raccordement correspondant de la canalisation d'alimentation au patient. Ainsi, la mise en place du corps d'embase (4) avec sa cartouche filtre-clapet (70) provoque le déplacement vers l'arrière de la cartouche (60) du clapet d'embase et ce contre l'action du ressort de compression correspondant (62), ce qui provoque la désactivation du clapet d'embase. Par ailleurs, la connexion du raccordement de la canalisation au patient sur l'extrémité avant du corps d'embase, provoque le déplacement vers l'arrière de l'ensemble clapet (6) et filtre (7), afin de permettre la distribution du fluide médical.

Préférentiellement, l'embase (2) est en laiton. tandis que la portion de tuyauterie rapportée (1), qui est en cuivre, est associée par brasage sur la portion de tuyauterie (20), afin de permettre un raccordement par brasure ultérieurement sur le circuit de fluides (3).

On a compris que lorsque le corps d'embout (4) est monté sur l'embase murale (2), le clapet d'embase (6) et le filtre (7) avec sa cartouche sont aligné et en contact l'un avec l'autre pour être sollicité ensemble vers l'avant et assurer ensemble l'étanchéité de la prise (1).

On a compris aussi que lorsque le corps d'embout (4) est désaccouplé de l'embase murale (2), ladite embase murale est totalement étanche grâce à son clapet d'embase (6) qui est sollicité vers l'avant de façon à ce que le joint torique (61) soit en appui sur son siège (63) et empêcher ainsi toute fuite. Grâce à l'invention qui vient d'être décrite et à son procédé de fabrication, de nombreux avantages sont obtenus comme la facilité d'usinage de chacune des pièces ainsi que la simplification du flux d'usinage, la réduction des encours, la réduction des coûts, l'interchangeabilité avec le matériel existant, chacune des parties ne recevant que les traitements lui étant propres et utiles.

Un avantage réside également dans la possibilité de changer un embout défectueux sans intervenir sur le réseau et adapter un embout spécifique sur une base standard.

## Revendications

1. Prise médicale (1) destinée à la mise en oeuvre de fluides médicaux afin d'effectuer des branchements rapides d'une fiche d'un flexible d'un appareil, sans risque d'inversion des fluides, du type comprenant une embase de prise murale (2) constituant un point de sortie ou d'entrée dans le circuit de distribution et destinée à être raccordée sur une tuyauterie (3), notamment par brasure, laquelle embase (2) se prolonge par un corps d'embout (4), l'ensemble renfermant une partie fonctionnelle (5) formée par un clapet d'embase (6) et d'un filtre avec clapet (7), spécifiques et adaptés aux fluides, **caractérisée en ce que** ladite prise (1) est réalisée en deux parties distinctes, dont une première partie est formée par l'embase de prise murale (2) connectée à une tuyauterie d'alimentation, et une seconde partie formée par le corps d'embout (4) l'une et l'autre des parties comportant des moyens de raccordement complémentaires, de manière à permettre une interchangeabilité de la seconde partie sans intervention sur la première, pour remplacement ou en fonction du fluide à distribuer.

2. Prise médicale selon la revendication 1, **caractérisée en ce que** le corps d'embout (4) est fixé de façon amovible sur l'embase murale correspondante (2) par vissage

3. Prise médicale selon la revendication 1 ou 2, **caractérisée en ce que** les moyens de raccordement complémentaires sont constitués d'une part par un filetage interne (8) réalisé sur l'alésage (9) d'une extrémité ouverte (10) de l'embase (2) opposée à une extrémité (11) formant un fond, et d'autre part par un filetage externe (12) réalisée à une extrémité (13) du corps d'embout (4), apte à coopérer en vissage avec le filetage (8) de l'embase (2).

4. Prise médicale selon la revendication 1 ou 2 **caractérisée en ce que** le corps d'embout (4) comprend selon son axe général un clapet d'embase (6) et un filtre avec clapet (7), spécifiques adaptés aux fluides.

5. Prise médicale selon l'une quelconque des revendications 1 à 4, **caractérisée en ce que** le corps d'embout (4) est traversé de part en part par un logement axial (14) présentant des diamètres étagés, dont une extrémité (15) dirigée vers l'embase (2) est d'un diamètre réduit par rapport au logement (14) et filetée intérieurement pour permettre le vissage d'un clapet choisi (6) et dont l'autre extrémité (16) opposée est de diamètre élargi par rapport audit logement (14) et filetée intérieurement pour permettre le vissage d'un filtre choisi (7), pour constituer ainsi un sous-ensemble spécifique et indépendant de l'embase (2), ne constituant qu'un élément de tuyauterie standard et inamovible.

6. Prise médicale selon l'une des revendications 1 à 4, **caractérisée en ce que** l'embase (2) forme un logement axial borgne (17) dans lequel débouche un trou circulaire (18) traversant radialement la paroi périphérique (19) de l'embase (2) et sur lequel trou (18) est rapportée par brasage une portion de tuyauterie (20), destinée à être raccordée ultérieurement à un circuit de fluide (3).

7. Prise médicale selon la revendication 4, **caractérisée en ce que** l'embase (2) est en laiton et subit une opération de chromage superficielle, et **en ce que** la portion de tuyauterie rapportée (20) est en cuivre, dont au moins une partie (20a) n'est pas chromée, afin de permettre un raccordement par brasure sur le circuit de fluide (3).

8. Prise médicale selon l'une quelconque des revendications 1 à 3, **caractérisée en ce que** le corps d'embout (4) comprend selon son axe général un filtre avec clapet (7) spécifiques adaptés aux fluides, tandis que l'embase murale (2) comprend un clapet (6) logé dans une cartouche d'embase (60).

9. Prise médicale selon la revendication 8, **caractérisée en ce que** la cartouche d'embase (60) comprend un joint torique d'étanchéité (61), tandis qu'il est sollicité vers l'avant en position de fermeture grâce à un ressort de compression conique (62), ladite position de fermeture étant celle ou le joint torique d'étanchéité (61) est en appui sur son siège d'appui (63).

10. Prise médicale selon la revendication 9, **caractérisée en ce que** r le filtre-clapet (7), est constitué par une cartouche (70) comprenant un joint torique d'étanchéité (71), tandis que la cartouche est sollicitée vers l'avant en position de fermeture grâce à un ressort de compression conique (72), ladite position de fermeture étant celle ou le joint torique d'étanchéité (71) est en appui sur son siège d'appui (73).

11. Prise médicale selon l'une quelconque des revendications précédente **caractérisée en ce que** le filtre avec clapet (7) du corps d'embase est aligné et en appui sur le clapet d'embase (6).

12. Prise médicale selon l'une quelconque des revendications précédentes, **caractérisée en ce que** l'embase murale (2) comprend une plaque de base (200) permettant sa fixation sur un mur (100) par exemple par des vis (101).

13. Prise médicale selon la revendication 12, **caractérisée en ce que** la plaque de base (200), ainsi que la portion de tuyauterie (20) sont solidaires de l'embase murale (2) pour ne former qu'une seule et même pièce .
